Europäisches Patentamt

European Patent Office

Office européen des brevets

(11) Veröffentlichungsnummer: **0 228 511**
A2·

(12) **EUROPÄISCHE PATENTANMELDUNG**

(21) Anmeldenummer: 86113525.9

(22) Anmeldetag: 01.10.86

(51) Int. Cl.⁴: **A 61 F 2/32**

(30) Priorität: 02.10.85 DE 3535158

(43) Veröffentlichungstag der Anmeldung:
15.07.87 Patentblatt 87/29

(84) Benannte Vertragsstaaten:
AT CH ES FR GB IT LI NL SE

(71) Anmelder: GMT Gesellschaft für medizinische Technik
mbH
Holstenstrasse 2
D-2000 Hamburg 50(DE)

(71) Anmelder: Waldemar Link GmbH & Co
Barkhausenweg 10
D-2000 Hamburg 63(DE)

(72) Erfinder: Nieder, Elmar
Hinterdeich 117
D-2155 York(DE)

(72) Erfinder: Engelbrecht, Eckart
Andreasstrasse 33
D-2000 Hamburg 60(DE)

(72) Erfinder: Keller, Arnold
An der Naherfurth
D-2061 Kaihude(DE)

(74) Vertreter: Heldt, Gert, Dr. Dipl.-Ing.
Neuer Wall 59 III
D-2000 Hamburg 36(DE)

(54) Sattelprothese.

(57) Eine Sattelprothese dient zum Ersatz eines an einem Beckenknochen auszubildenden das Hüftgelenk ersetzenden Gelenkes. Diese Sattelprothese weist zwei einen Sattel zwischen sich ausbildende Sattelhörner auf, die sich im Bereich des Sattels zu einem sich in Längsrichtung erstreckenden Zylinderstück vereinigen. Das Zylinderstück mündet in einen in einen Oberschenkelknochen einzusetzenden Schaft, zwischen dem und dem Zylinderstück ein Kragen ausgebildet ist, der sich auf einem dem Sattel zugewandten oberen Ende des Oberschenkelknochens abstützt. Der Sattel ist in seiner Lage zwischen dem oberen Abschluß des Oberschenkelknochens einerseits und dem Beckenknochen andererseits einstellbar angeordnet. Das Zylinderstück und der Schaft sind als zwei voneinander getrennte Bauteile ausgebildet, die gegeneinander einstellbar angeordnet sind. Am Zylinderstück ist ein sich in Richtung des Schaftes erstreckender Bolzen befestigt, der in eine entsprechende Bohrung hineinragt, die sich in Längsrichtung des Schaftes durch diesen erstreckt. Der Bolzen ist in voneinander verschiedenen Schwenkstellungen im Schaft justierbar angeordnet.

./...

Fig. 1

Die Erfindung betrifft eine Sattelprothese zum Ersatz eines an einem Beckenknochen auszubildenden das Hüftgelenk ersetzenden Gelenkes mit zwei einen Sattel zwischen sich ausbildenden Sattelhörnern, die sich im Bereich des Sattels zu einem sich in Längsrichtung erstreckenden Zylinderstück vereinigen, das in einen in einen Oberschenkelknochen einzusetzenden Schaft einmündet, zwischen dem und dem Zylinderstück ein Kragen ausgebildet ist, der sich auf einem dem Sattel zugewandten oberen Ende des Oberschenkelknochens abstützt.

Derartige Sattelprothesen haben sich in der Praxis, insbesondere in den Fällen gut bewährt, in denen aufgrund von Knochenverlusten im Bereich des Beckens, insbesondere der natürlichen Hüftpfanne, ein Hüftgelenk im Beckenknochen nicht mehr befestigt werden kann. In diesen Fällen kann die Sattelprothese Verwendung finden. Diese ragt mit einem ihrer Sattelhörner durch eine im Beckenknochen vorhandene Öffnung hindurch, so daß der Beckenknochen sich mit einer Kante im Bereich der Öffnung im Sattel abstützen kann. Dabei wird der Sattel meist in der Region eines zuvor im Beckenknochen ausgebildeten Pfannendaches oder bezüglich einer von einem im Beckenknochen schwenkbar zu lagernden Oberschenkel abgewandten Richtung oberhalb des Pfannendaches abgestützt. Darüber hinaus können jedoch beliebige andere Stellen des Beckenknochens zur Lagerung des Sattels benutzt werden. Je nach der Abweichung der Lagerung aus dem Bereich eines zuvor im Beckenknochen befestigten Hüftgelenkes ist eine Gestaltung der Sattelprothese erwünscht, die eine Anpassung der Sattelprothese an die geometrisch relativ komplizierte Gestaltung des Beckenknochens erlaubt.

Aufgabe der vorliegenden Erfindung ist es daher, die Sattelprothese der einleitend genannten Art so zu verbessern, daß sie sich verschiedenen Gestaltungen von Beckenknochen einerseits und von Anlenkungen an verschiedenen Stellen eines Beckenknochens andererseits anpassen kann.

Diese Aufgabe wird erfindungsgemäß dadurch gelöst, daß der Sattel in seiner Lage zwischen dem oberen Ende des Oberschenkelknochen einerseits und dem Beckenknochen andererseits einstellbar angeordnet ist.

Durch die Einstellbarkeit kann die Sattelprothese nicht nur an die individuellen Verhältnisse angepaßt werden, die bei ihren jeweiligen Trägern vorgefunden werden. Vielmehr kann auch an einem bestimmten Beckenknochen die Abstützung des Oberschenkels so vorgenommen werden, daß ein für die Abstützung am besten geeigneter Teil des Beckenknochens für die Abstützung Verwendung findet. Zwar ist der Sattel geeignet, aufgrund seiner Gestaltung eine Vielzahl verschiedener Anwendungsfälle abzudecken, ohne daß eine besondere Einstellung des Sattels bezüglich des Beckenknochens notwendig erschiene. Im Sinne der von der Sattelprothese auszuübenden Funktion ist aber eine Anlenkung der Sattelprothese an den Beckenknochen erwünscht, bei der der Sattel eine der Form des Beckenknochens angepaßte Höhen- und Seitenlage aufweist, damit eine optimale Funktion der Sattelprothese gewährleistet ist. Eine derartige Anpassung legt in besonders günstiger Weise die Lage des Oberschenkelknochens, der am Beckenknochen zu lagern ist, bezüglich des Beckenknochens fest. Auf diese Weise kann davon ausgegangen werden, daß der

Sattel bezüglich des Beckenknochens gleichbleibende und zuvor weitgehend festgelegte Bewegungen durchführt, die einerseits sehr genau vom Träger der Endoprothese gesteuert werden können und andererseits dem Träger der Endoprothese so gut wie keine Schwierigkeiten bereitet. Diese Schwierigkeiten könnten sich insbesondere daraus ergeben, daß die Sattelhörner in ihrem jeweiligen Einflußbereich eine ihrem Bewegungsablauf entsprechende Bewegungsfreiheit besitzen, ohne daß sie mit anderen am Beckenknochen vorhandenen Organen kollidieren. Zum anderen ist es jedoch auch notwendig, eine möglichst exakte Auflagefläche des Sattels am Beckenknochen einzuhalten.

Gemäß einer bevorzugten Ausführungsform der Erfindung sind das Zylinderstück und der Schaft als zwei voneinander getrennte Bauteile ausgebildet, die gegeneinander einstellbar angeordnet sind. Auf diese Weise ist es möglich, das Zylinderstück gegenüber dem Schaft in einer Weise anzuordnen, die dem jeweiligen individuellen Anwendungsfall am besten gerecht wird. Insbesondere ist eine Lagerung denkbar, bei der das Zylinderstück in den Schaft mehr oder minder weit abgesenkt werden muß, um Höhendifferenzen ausgleichen zu können, die im Bereich des Beckenknochens auftreten können. Darüber hinaus kann aber auch das Zylinderstück innerhalb des Schaftes verschwenkt werden und auf diese Weise sichergestellt werden, daß der Sattel eine Ebene aufspannt, die möglichst senkrecht zu einer Ebene verläuft, in der sich der Schaft und damit der Oberschenkelknochen in ihren Hauptbewegungsrichtungen bewegen.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

Fig. 1 : eine teilweise geschnittene Seitenansicht einer in einen Beckenknochen eingesetzten Sattelprothese,

Fig. 2 : eine teilweise geschnittene Seitenansicht einer anderen Sattelprothese,

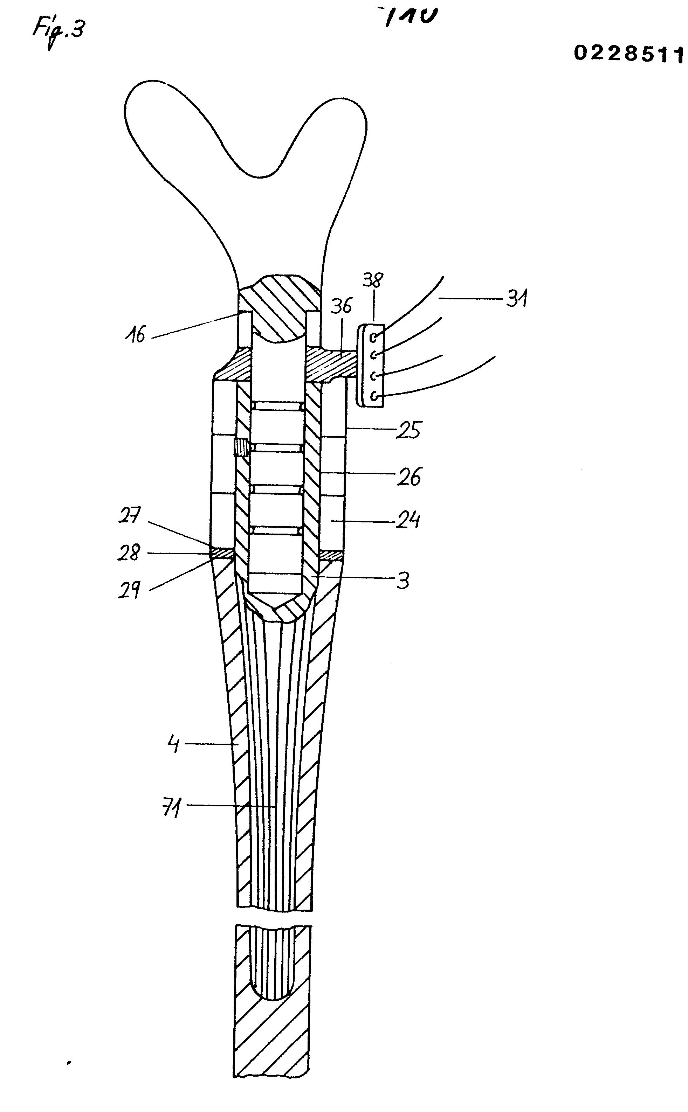

Fig. 3 : eine teilweise geschnittene Seitenansicht einer weiteren Sattelprothese,

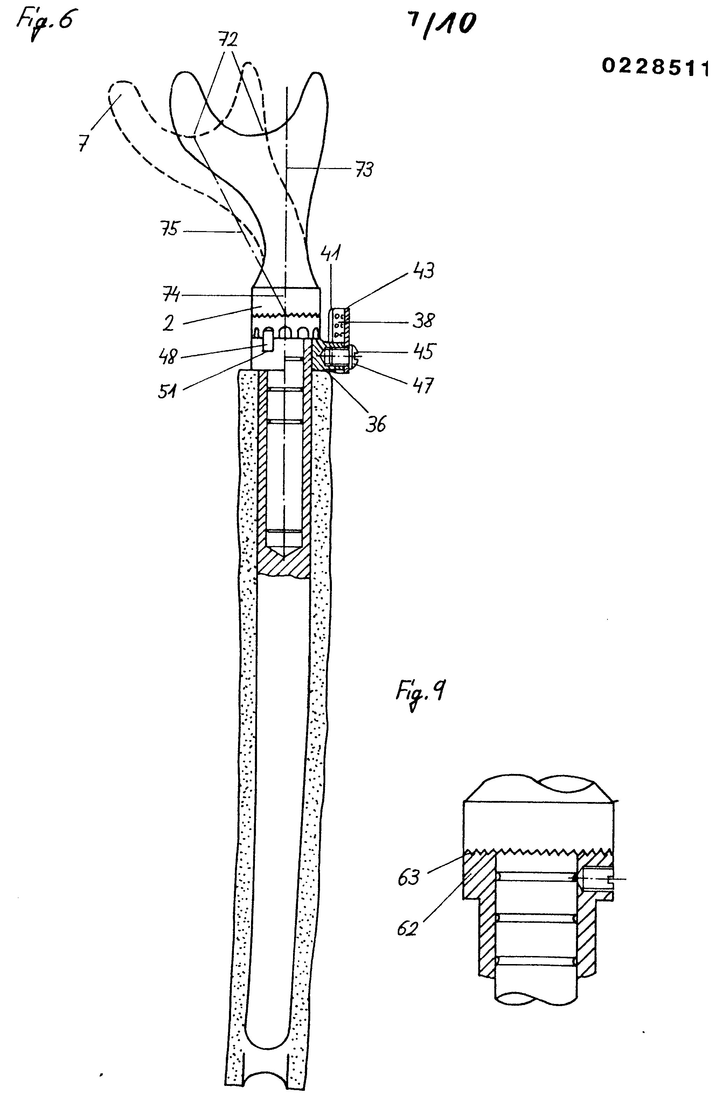

Fig. 4 : einen Querschnitt durch einen Schaft einer Sattelprothese entlang der Schnittlinie IV-IV in Figur 1,

Fig. 5 : ein Querschnitt einer anderen Sattelprothese entlang der Schnittlinie V-V in Figur 2,'

Fig. 6 : eine teilweise geschnittene Seitenansicht einer weiteren Sattelprothese mit zwei voneinander verschieden geneigten Sätteln,

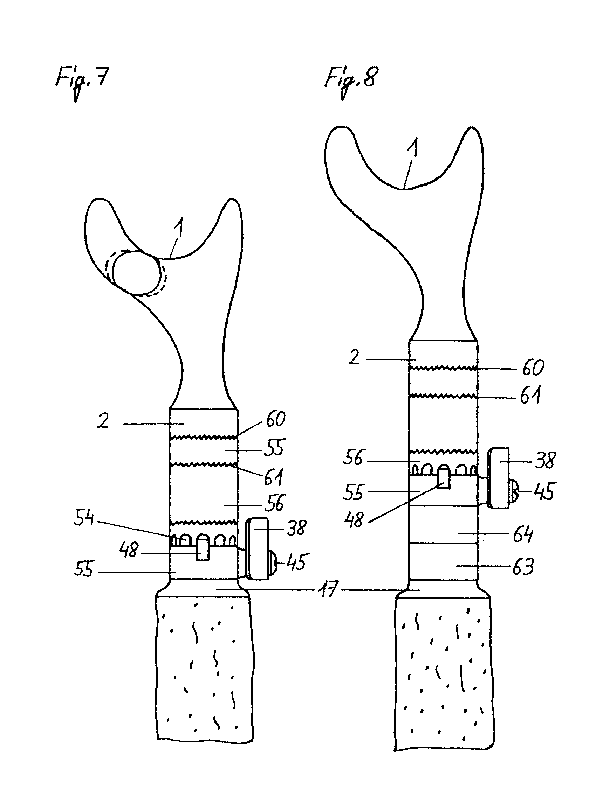

Fig. 7 : eine Seitenansicht einer Sattelprothese mit gezähnelten Distanzringen,

Fig. 8 : eine Seitenansicht einer Sattelprothese mit gezähnelten und ungezähnelten Distanzringen,

Fig. 9 : eine teilweise geschnittene Seitenansicht eines Übergangs zwischen einem Schaft und einem Zylinderstück,

0228511

-5-

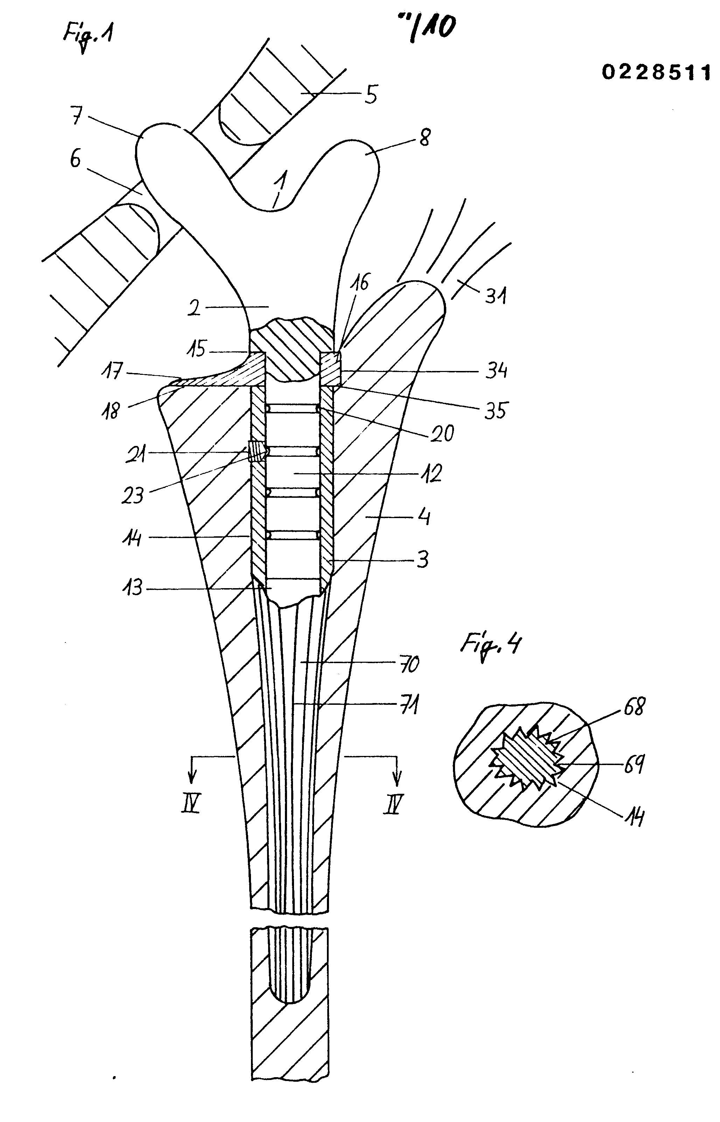

Fig. 1o : einen Längsschnitt durch einen Distanzring,

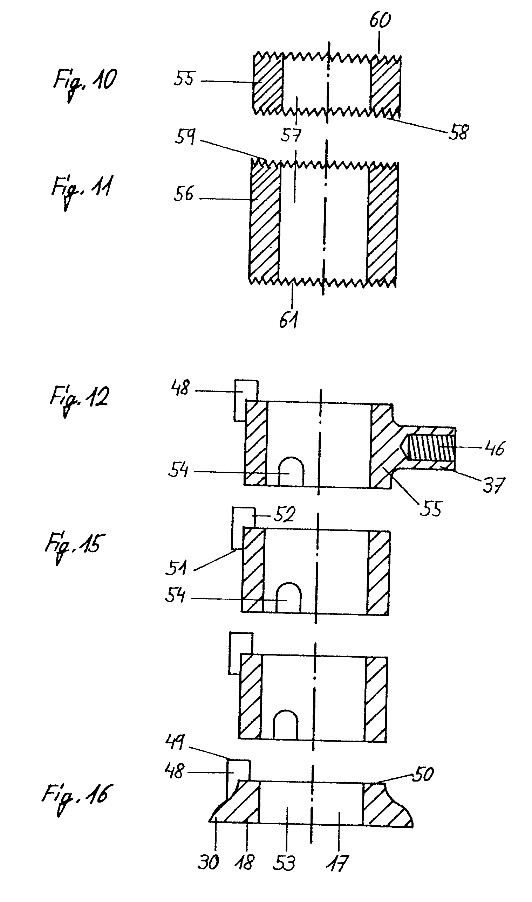

Fig. 11 : einen Längsschnitt durch einen anderen Distanzring,

Fig. 12 : einen Längsschnitt durch einen mit einem Ansatz versehenen Distanzring,

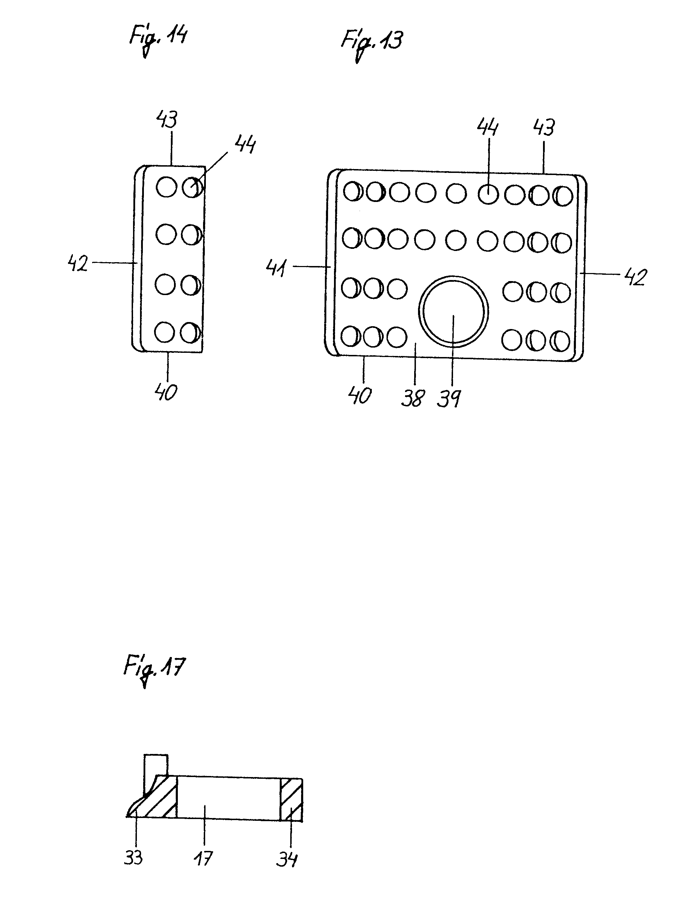

Fig. 13 : eine Draufsicht auf eine mit einem Distanzring verbindbare Trochanterscheibe,

Fig. 14 : eine Seitenansicht einer Trochanterscheibe,

Fig. 15 : einen Längsschnitt durch einen anderen Distanzring,

Fig. 16: einen Längsschnitt durch einen symmetrischen Kragen,

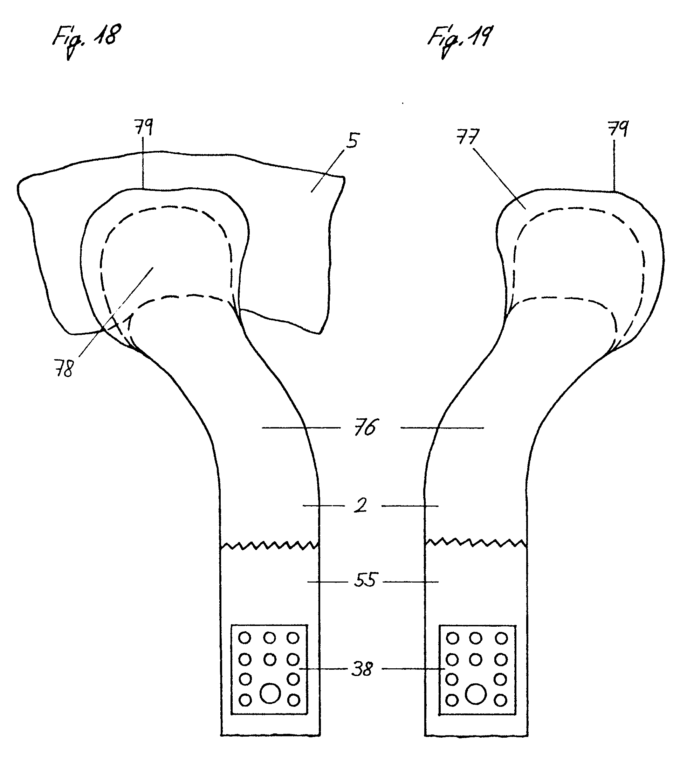

Fig. 17 : einen Längsschnitt durch einen anderen asymmetrischen Kragen,

Fig. 18 : eine Seitenansicht eines vorwärts geneigten Sattels,

Fig. 19 : einen Seitenansicht eines rückwärts geneigten Sattels,

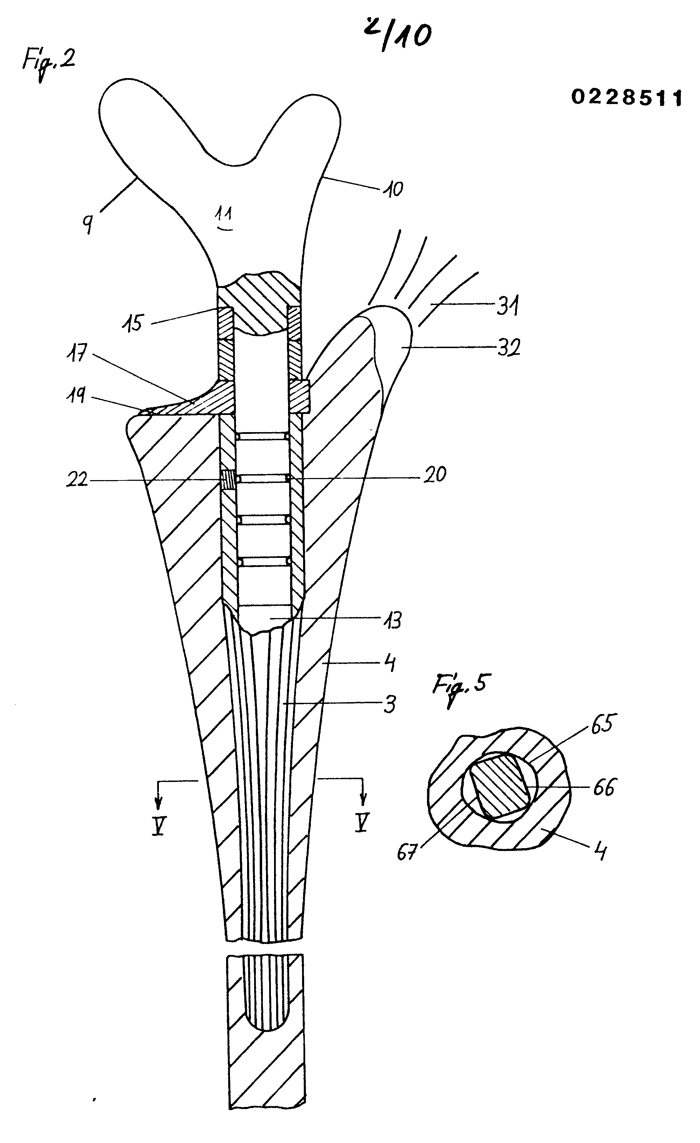

Fig. 2o : eine Draufsicht auf einen Sattel,

Fig. 21 : eine Draufsicht auf einen anderen Sattel,

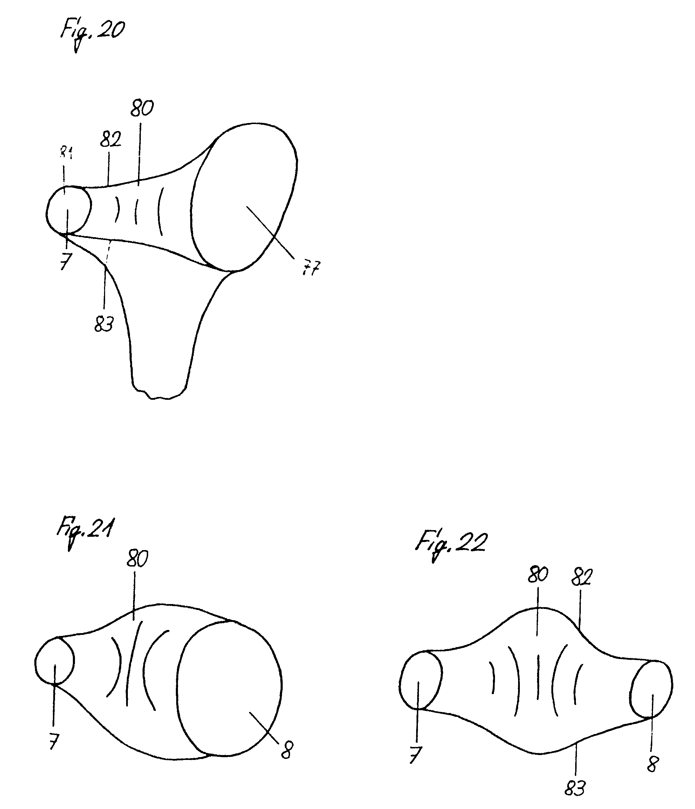

Fig. 22 : eine Draufsicht auf einen weiteren Sattel und

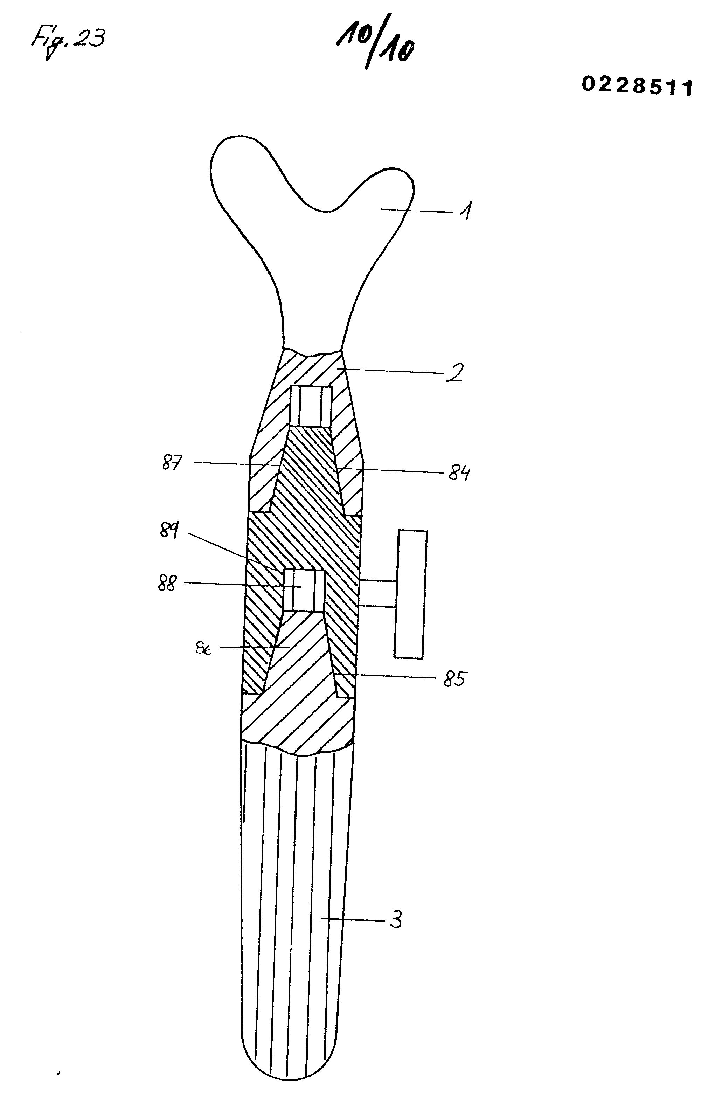

Fig. 23 : eine Seitenansicht einer Sattelprothese.

Eine Sattelprothese besteht im wesentlichen aus einem Sattel 1, einem Zylinderstück 2 und einem Schaft 3. Der Schaft 3 erstreckt sich in Längsrichtung durch einen Oberschenkelknochen 4, der über die Sattelprothese in einem Beckenknochen 5 verschwenkbar gelagert ist. Zu diesem Zwecke ist im Beckenknochen 5 eine Öffnung 6 vorgesehen, durch die sich der Sattel 1 mit einem seiner beiden ihn bildenden Sattelhörner 7, 8 erstreckt. Der Sattel 1 stellt sich als eine zwischen den beiden Sattelhörnern 7, 8 ausgebildete Mulde dar, die sich in Richtung auf das Zylinderstück 2 erstreckt, das zwischen dem Sattel 1 und dem Schaft 3 angeordnet ist. In das Zylinderstück 2 münden die beiden Sattelhörner 7, 8 mit ihren dem Sattel 1 abgewandten äußeren Begrenzungen 9, 1o ein. Darüber hinaus wird das Zylinderstück 2 voneinander gegenüberliegenden Seitenflächen 11 gebildet, die von den äußeren Begrenzungen 9, 1o der Sattelhörner 7, 8 eingeschlossen sind.

Von dem Zylinderstück 2 erstreckt sich in Richtung auf den Schaft 3 ein Bolzen 12, der in eine entsprechend vorgesehene Bohrung 13 hineinragt, die sich in etwa parallel zur Außenfläche 14 des Schaftes 3 mittig durch diesen erstreckt. Der Bolzen 12 mündet darüber hinaus auch mittig in das Zylinderstück 2 ein. Sein Durchmesser ist jedoch geringer als der des Zylinderstückes 2, so daß an der Einmündung des Bolzens 12 in das Zylinderstück 2 ein Rezeß 15 vorgesehen ist, um den das Zylinderstück 2 über den Bolzen hinauskragt. Mit diesem Rezeß 15 liegt das Zylinderstück 2 auf einer Auflagefläche 16 auf, die an einem Kragen 17 vorgesehen sein kann. Dieser Kragen 17 stützt sich mit seiner der Auflagefläche 16 abgewandten Unterfläche 1C auf einem für diese Zwecke vorgesehenen oberen Abschluß 19 des Oberschenkelknochens 4 ab. Es ist jedoch auch möglich, daß der Rezeß 15 sich unmittelbar auf dem oberen Abschluß 19 des Oberschenkelknochens 4 abstützt.

In dem Bolzen 12 sind eine Anzahl von Querrillen 2o vorgesehen, die sich im wesentlichen quer zur Längsrichtung des Bolzens 12 erstrecken. In eine dieser Querrillen 2o ragt eine Madenschraube 21 hinein, die sich in einem Gewindeloch 22 bewegt, das sich quer zur Längsrichtung des Schaftes 3 durch diesen in Richtung auf die Bohrung 13 erstreckt. Je nach der Höhe, in der sich die Öffnung 6 innerhalb des Beckenknochens 5 befindet, wird der Bolzen 12 mehr oder minder weit aus der Bohrung 13 in Richtung auf den Beckenknochen 5 herausgezogen und mit Hilfe der Madenschraube 21 innerhalb des Schaftes 3 befestigt. Zu diesem Zwecke ragt die Madenschraube 21 mit ihrer in Richtung auf den Bolzen 12 gerichteten Spitze 23 in eine der parallel zueinander verlaufenden Querrillen 2o hinein. Diese Querrillen 2o gestatten eine Verschwenkung des Bolzens 12 innerhalb der Bohrung 13, so daß bei einer im Oberschenkelknochen 4 gleichbleibenden Verankerung des Schaftes 3 der Sattel 1 in eine Lage verschwenkt werden kann, in der er am besten geeignet ist, den Beckenknochen 5 zwischen den beiden Sattelhörnern 7, 8 aufzunehmen. Statt der Querrillen 2o können auch eine Anzahl von Sacklöchern in die Oberfläche des Bolzens 12 eingebracht werden, die auf einer bezüglich des Rezeß 15 gleichbleibenden Ebene unmittelbar nebeneinander angeordnet sind und der Aufnahme der Spitze 23 dienen.

Der Sattel 1 wird hinsichtlich seiner Höhe, um die er sich oberhalb des oberen Abschlusses 19 aus dem Oberschenkelknochen 4 erhebt, jedoch nicht nur mit Hilfe der Madenschraube 1 festgelegt. Vielmehr sind zwischen dem oberen Abschluß 19 und der Unterfläche 18 des Kragens 17 Abstandsringe 24 vorgesehen, deren Höhe und Anzahl sich danach richtet, wie weit der Bolzen 12 aus der Bohrung 13 herausgezogen werden muß, um den Sattel 1 in der Öffnung 6 mit dem Beckenknochen 5 zur Anlage bringen zu können. Diese Abstandsringe 24 können aus Polyäthylen oder Metall bestehen. Darüber hinaus ist es möglich, die Abstandsringe 24 als Polyurethan, Apathith oder Keramik oder einem anderen bioaktiven Material herzustellen. Ihr äußerer Umfang 25 richtet sich nach einem vom Oberschenkelknochen 4 vorgegebenen Querschnitt, dessen Größe er besitzt. Durch die Abstandsringe 24 erstreckt sich eine Bohrung 26, deren Querschnitt dem Querschnitt des Schaftes 3 entspricht, so daß sich die Abstandsringe 24 leicht aber ohne sehr großes Spiel über den Schaft 3 schieben lassen. Ein dem Sattel 1 abgewandter unterer Abstandsring 24 liegt mit seiner dem Oberschenkelknochen 4 zugewandten Unterkante 27 auf einem Metallring 28 auf, der mit seiner dem unteren Abstandsring 24 abgewandten unteren Auflagefläche 29 auf dem oberen Abschluß 19 des Oberschenkelknochens 4 aufliegt. Dabei können sowohl auf der unteren Auflagefläche 29 als auch auf dem oberen Abschluß 19 Erhöhungen beziehungsweise Vertiefungen vorgesehen sein, die ineinander greifen und einen Formschluß zwischen der unteren Auflagefläche 29 und dem oberen Abschluß 19 herbeiführen. Über den Metallring 28 werden die von den Abstandsringen 24 übertragenen Druckkräfte in den Oberschenkelknochen 4 eingeleitet.

Die Anzahl und Höhe der Abstandsringe 24 bestimmt den Abstand, den der Kragen 17 vom oberen Abschluß 19 des Oberschenkelknochens 4 einhält, soweit der Kragen 17 mit seiner Unterfläche 18 nicht unmittelbar auf dem oberen Abschluß 19 des Oberschenkelknochens 4 aufliegt. Dieser Kragen 17 kann als ein symmetrischer Kragen ausgebildet sein, der einen in Richtung auf den Oberschenkelknochen sich ausweitenden Wulst 30 aufweist. Dieser Wulst 30 endet in der Unterfläche 18, so daß diese über eine vergleichsweise große Fläche die von dem Sattel 1 auf den Oberschenkelknochen 4 zu übertragenden Kräfte in den oberen Abstandsring 24 beziehungsweise den oberen Abschluß 19 des Oberschenkelknochens 4 einleiten kann. Ein derartiger symmetrischer Wulst 30 wird jedoch nur bei solchen Kragen 17 verwendet, die zum Einsatz gelangen, wenn ein für den Anschluß von Muskeln 31 vorgesehener Knochenteil, der Trochantermajor 32 nicht mehr vorhanden ist oder aus medizinischen Gründen nicht mehr erhalten werden kann. In diesen Fällen wird der obere Abschluß 19 als eine sich quer zur Längsrichtung des Oberschenkelknochens 4 auf dessen gesamten Umfang sich erstreckende Auflagefläche ausgebildet, auf der der Kragen 17 sich symmetrisch abstützt. Sollte sich indessen herausstellen, daß der Trochantermajor 32 noch so gut erhalten ist, daß er für den Anschluß der Muskeln 31 dienen kann, so wird der Kragen 17 mit einem unsymmetrischen Wulst 33 versehen, der sich ausschließlich in eine vom Trochantermajor 32 abgewandte Richtung erstreckt, so daß im Bereich des Trochanters Major 32 lediglich eine zylindrisch verlaufende Hinterkante 34 den Kragen 17 begrenzt. Im Bereich dieser Hinterkante 34 stützt sich der Kragen 17 im wesentlichen auf dem Schaft 3 und gegebenenfalls mit einem sehr geringem Teil 35 seiner Unterfläche 18 auf einer entsprechend vorgesehenen Auflagefläche des Oberschenkelknochens 4 ab.

Ist der Trochantermajor 32 nicht mehr erhalten, so kann am Kragen 17 eine Halterung 36 befestigt sein, die einer Anlenkung der Muskeln 31 am Oberschenkelknochen 4 beziehungsweise der Sattelprothese dient. Diese Halterung 36 kann als ein Zapfen 37 ausgebildet sein, auf dem eine Trochanterplatte 38 befestigt ist. Diese Trochanterplatte 38 ist mit einer Aufnahmebohrung 39 versehen, deren Querschnitt dem Querschnitt des Zapfens 37 entspricht und auf diesen aufgeschoben werden kann. Diese Bohrung 39 ist im Regelfall an einer dem Oberschenkelknochen 4 zugewandten Unterkante 40 der Trochanterplatte 38 vorgesehen, so daß diese sich im wesentlichen in Richtung auf den Sattel 1 erstreckt. Die Aufnahmebohrung 39 ist in etwa mittig zu Seitenkanten 41, 42 der Trochanterplatte 38 vorgesehen, die einander in etwa parallel verlaufen. Bezüglich einer sich durch die Mitte der Aufnahmebohrung 39 erstreckenden Mittellinie ist die Trochanterplatte 38 zylindrisch gewölbt, so daß sowohl die Unterkante 40 als auch eine ihr gegenüberliegende Oberkante 43 in Form von Kreisbögen gewölbt sind. Aufgrund dieser Wölbung verläuft die Trochanterplatte 38 im Abstand des Zapfens 37 schildförmig vor dem Zylinderstück 2, so daß die Seitenkanten 41, 42 in Richtung auf das Zylinderstück 2 gegenüber der durch die Aufnahmebohrung 39 verlaufenden Mittellinie zurückgewölbt sind. In der Trochanterplatte 38 ist eine Vielzahl von Verklammerungsbohrungen 44 vorgesehen, die über eine von der Trochanterplatte 38 vorgegebene Fläche verteilt sind. Die Verteilung kann symmetrisch oder asymmetrisch erfolgen. Zweckmäßigerweise sind die Verklammerungsbohrungen 44 in einander parallel verlaufenden Reihen angeordnet, in denen jeweils eine Verklammerungsbohrung 44 auf die andere folgt. Lediglich im Bereich der Aufnahmebohrung 39 ist die Anzahl der Verklammerungsbohrungen 44 so bemessen, daß die Aufnahmebohrung 39 in eine Fläche eingebracht werden kann, die so groß ist, daß sie unter dem Einfluß der von den Muskeln 31 in den Oberschenkelknochen 4 einzuleitenden Kräfte nicht aus der Trochanterplatte 38 ausbrechen kann.

Die mit ihrer Aufnahmebohrung 39 auf den Zapfen 37 aufgeschobene Trochanterplatte 38 wird mit Hilfe einer Schraube 45 auf dem Zapfen 37 befestigt. Zu diesem Zwecke ist in dem Zapfen 37 ein Gewinde 46 eingebracht, in das die Schraube 45 eingeschraubt wird, die mit ihrem Kopf 47 die Trochanterplatte 38 auf dem Zapfen 37 festhält.

An dem Kragen 17 kann ein Zapfen 48 vorgesehen sein, der sich mit seiner Längsachse parallel zur Längsachse des Bolzens 12 erstreckt. Dieser Zapfen 48 ragt mit seinem der Unterfläche 18 des Kragens 17 abgewandten oberen Ende 49 über eine der Unterfläche 18 abgewandte Oberfläche 5o des Kragens 17 hinaus. Der Zapfen 48 kann beispielsweise als ein Zylinder ausgebildet sein, der mit seinem dem oberen Ende 49 abgewandten unteren Ende 51 am Kragen 17 befestigt ist. Diese Befestigung erfolgt in der Weise, daß der Zapfen 48 mit seiner dem Bolzen 12 zugewandten Oberfläche 52 in unmittelbarer Nachbarschaft einer Bohrung 53 verläuft, die sich durch den Kragen 17 erstreckt und den Bolzen 12 aufnimmt. Bei einer zylindrischen Ausbildung des Zapfens 48 ragt dieser mit seinem unteren Ende 51 etwa bis zu seiner Mittellinie in den Kragen 17 hinein, während der übrige Teil des unteren Endes 51 aus dem Kragen 17 herausragt. Das obere Ende 49 des Zapfens 48 ragt in eine den Abmessungen des Zapfens 48 entsprechende Ausnehmung 54 hinein, die am Zylinderstück 2 vorgesehen ist. Dabei ragt der Zapfen 48 bis etwa zu seiner Mittellinie in die Ausnehmung 54 mit seinem oberen Ende 49 hinein, während der übrige Teil des oberen Endes 49 aus dem Zylinderstück 2 herausragt. Auf diese Weise ist dafür gesorgt, daß sich der Kragen 17 gegenüber dem Bolzen 12 selbst dann nicht verdrehen kann, wenn relativ große Kräfte über die Muskeln 31 in den Kragen 17 eingeleitet werden.

Das Zylinderstück 2 kann aus einer Vielzahl von mehr oder minder breiten Distanzringen 55, 56 bestehen, die mit einer Mittelbohrung 57 versehen sind. Durch diese Mittelbohrung 57 erstreckt sich der Bolzen 12. Die Distanzringe 55, 56 können an ihren einander zugewandten Auflageflächen 58, 59 mit einer Zähnelung 6o versehen sein, so daß sie untereinander in einer vorge-

–12–

gebenen Lage fixiert werden können. Darüber hinaus kann eine Zähnelung 61 am Rezeß 15 vorgesehen sein, mit dem das Zylinderstück 2 über den Bolzen 12 hinausragt. Mit Hilfe dieser Zähnelung 61 kann auch die Lage der Distanzringe 55, 56 gegenüber dem Sattel 1 festgelegt werden. Schließlich ist es denkbar, eine Zähnelung 63 auch unmittelbar an einem dem Zylinderstück 2 zugewandten Ende 62 des Schaftes 3 vorzusehen. Auf diese Weise kann das Zylinderstück 62 unmittelbar gegenüber dem Schaft 3 ausgerichtet werden.

Zwischen den Distanzringen 55, 56 können Zapfen 48 vorgesehen sein, die in ähnlicher Weise die Lage der Distanzringe 55, 56 festlegen, wie der Zapfen 48 die Lage des Kragens 17 gegenüber den Distanzringen 55, 56 festlegt. Zu diesem Zwecke können an den einzelnen Distanzringen Zapfen 48 und entsprechende Ausnehmungen 54 vorgesehen sein. Schließlich ist es denkbar, die Trochanterplatte 38 auch an einem der Distanzringe 55 zu befestigen. In diesem Falle ist der Zapfen 37 an dem Distanzring 55 befestigt. Zweckmäßigerweise wird der mit der Trochanterplatte 38 versehene Distanzring 55 mit einem benachbarten Distanzring 56 über einen Zapfen 48 verbunden, der in eine entsprechende Ausnehmung 54 des Distanzringes 56 hineinragt.

Der mit der Trochanterplatte 38 versehene Distanzring 55 kann sich unmittelbar in Richtung auf den Sattel 1 an den Kragen 17 anschließen. Es ist jedoch auch möglich, zwischen dem Kragen 17 und dem mit der Trochanterplatte 38 versehenen Distanzring 55 weitere Distanzringe 63, 64 vorzusehen. Zwischen diesen Distanzringen 63, 64 sowie zwischen dem Distanzring 63 und dem Kragen 17 können Oberflächen vorgesehen sein, die eine formschlüssige Verbindung zwischen den Distanzringen 63, 64 sowie zwischen diesen und dem Kragen 17 nicht erlauben. Ebenso kann auf formschlüssige Verbindungen zwischen dem Distanzring 64 und dem die Trochanterplatte 38 tragenden Distanzring 55 verzichtet werden. Die Distanzringe 55 können aus Metall oder Polyäthylen hergestellt werden. Es ist jedoch auch möglich, die Distanzringe 55 aus einem Polyurethan, Apathith, Keramik oder einem anderem bioaktiven Material herzustellen.

Mit Hilfe der Distanzringe 55, 56 einerseits und den Abstandringen 24 andererseits ist eine genaue Anpassung der Sattelprothese an die jeweiligen Gegebenheiten eines Beckenknochens 5 möglich. Dabei dienen die Abstandsringe 24 im wesentlichen dazu, die Höhe des Kragens 17 festzulegen. Auf die Höhe des Kragens 17 bezüglich des oberen Abschluβes 19 des Oberschenkelknochens 4 kommt es im wesentlichen dann an, wenn an dem Kragen 17 die Muskeln 31 angelenkt sind.

Demgegenüber dienen die Distanzringe 55, 56, 63, 64 dazu, den Abstand des Sattels 1 gegenüber dem Kragen 17 festzulegen. Dabei kann durch entsprechende Anordnung der Distanzringe 55, 56, 63, 64 die genaue Lage der Trochanterplatte 38 gegenüber dem Sattel 1 festgelegt werden.

Eine Verschwenkung des Sattels 1 gegenüber dem Schaft 3 ist dadurch möglich, daß der Bolzen 12 innerhalb der Bohrung 13 verschwenkt wird. Eine Festlegung erfolgt mit Hilfe der Madenschraube 21. Darüber hinaus kann jedoch auch die genaue Lage der Trochanterplatte 38 dadurch festgelegt werden, daß die Zähnelungen 60, 61, 63 einander so zugeordnet werden, daß die Trochanterplatte 38 einen genauen Anschluβ der Muskeln 31 ermöglicht. Schließlich sind über den Umfang des Zylinderstückes 2 verteilt eine Vielzahl von Ausnehmungen 54 vorgesehen. Durch eine genaue Auswahl einer dieser Ausnehmungen 54 wird die genaue Lage des mit der Trochanterplatte 38 versehenen Distanzringes 55 gegenüber dem benachbarten Distanzring 56 beziehungsweise des Kragens 17 gegenüber dem Zylinderstück 2 festgelegt.

Der Schaft 3 kann eine glatte Oberfläche 65 aufweisen.
Diese glatte Oberfläche 65 kann einen kreisförmigen
Querschnitt aufweisen. Es ist jedoch auch denkbar, zur
genauen Festlegung des Schaftes 3 innerhalb des Oberschenkelknochens 4 den Schaft 3 mit Führungsflächen 66,
67 zu versehen, die einander gegenüber liegen und planparallel verlaufen. Sie kommen an entsprechenden Auflageflächen zur Anlage, die innerhalb des Oberschenkelknochens 4 vorgesehen sind, so daß eine Verschiebung
des Schaftes 3 innerhalb des Oberschenkelknochens 4
verhindert wird. Zu diesem Zwecke kann der Schaft 3
auch eckige Querschnitte besitzen. Es ist beispielsweise denkbar, den Schaft mit einem viereckigen oder
dreieckigen Querschnitt zu versehen. In jedem Falle werden die Ecken mit einem kleinen Radius abgerundet. Im
Regelfall werden derartige Schäfte 3 mit Zement in den
Oberschenkelknochen 4 eingesetzt.

Darüber·hinaus ist es jedoch auch denkbar, den Schaft 3
mit einer gerillten Oberfläche 68 zu versehen, die als
Außenfläche 14 sich in eine entsprechende innere Oberfläche 69 des Oberschenkelknochens 4 einschneidet. In
der gerillten Oberfläche 68 sind Rillen 7o vorgesehen.
Diese erstrecken sich in Längsrichtung des Schaftes 3.
Zwischen zwei einander jeweils benachbarten Rillen 7o
ist eine scharfe Kante 71 vorgesehen, die sich beim Einsetzen des Schaftes 3 in den Oberschenkelknochen 4 in
eine bereits vorbereitete Knochenhöhle einschneidet. Auf
diese Weise wird eine genaue Festlegung des Schaftes 3
innerhalb des Oberschenkelknochens 4 insbesondere dann
herbeigeführt, wenn der Schaft 3 zementlos im Oberschenkelknochen 4 verankert wird. Es ist jedoch auch möglich,
den mit den Rillen 7o versehenen Schaft 3 unter Zuhilfenahme von Zement innerhalb des Oberschenkelknochens zu
verankern. Dabei kann der Zement in Form eines Granulates Verwendung finden, das Bestandteile enthält, mit deren Hilfe Entzündungen bekämpft beziehungsweise ihr Entstehen verhindert wird.

Die mit einem Knochen in Berührung kommenden Teile der Endoprothese können auf ihrer dem Knochen zugewandten Oberseite mit einer Beschichtung versehen werden, die eine bioaktive Substanz enthält, deren Vorhandensein das Anwachsen des Knochens an der Oberfläche der Endoprothese begünstigt. Insbesondere ist es möglich, auf der Oberfläche des Schaftes 3 eine Beschichtung mit Apathith beziehungsweise Keramik vorzusehen. Eine derartige Beschichtung kommt auch bei allen anderen Teilen in Betracht, die in einen Knochen hineinragen. Darüber hinaus ist es jedoch auch möglich, die ausschließlich mit einem Bindegewebe in Berührung kommenden Teile der Endoprothese mit einer Beschichtung aus Polyurethanschaum oder Velour zu versehen. Eine derartig beschichtete Oberfläche wird von den Bindegeweben durchwachsen, so daß diese mindestens einen vorübergehenden Halt an der Endoprothese finden. Beispielsweise ist es denkbar, in Richtung der auf die Trochanterplatte 38 sich ausdehnenden Bänder hinter dieser Trochanterplatte 38 eine mit einem dieser Materialien beschichtete Oberfläche vorzusehen, so daß die durch die Verklammerungsbohrungen 44 hindurchwachsenden Bänder hinter der Trochanterplatte 38 eine Aufnahme finden und auf diese Weise die Trochanterplatte 38 und damit die gesamte Endoprothese mit den Muskeln 31 verbinden. Schließlich kann beispielsweise der Schaft 3 auf seiner Oberfläche mit einer Vielzahl kleiner Kugeln oder anderer Erhebungen versehen werden. Diese verklammern sich beim Einsetzen des Schaftes 3 mit dem Oberschenkelknochen 4. Schließlich ist es auch

— 16 —

denkbar, dem Schaft 3 eine poröse Oberfläche zu geben, die ein Anwachsen des Knochens begünstigt.

Darüber hinaus können die Oberflächen der Sattelprothese, an denen ein Anwachsen des Bindegewebes beziehungsweise des Knochens begünstigt werden soll, auf ihren Oberflächen mit einem bioaktiven Material beschichtet werden. Beispielsweise können sowohl die Abstandsringe 24 als auch die Distanzringe 55, 56 mit einem Polyurethanschaum oder einem Velour zu diesem Zwecke beschichtet werden. Demgegenüber werden diejenigen Teile der Sattelprothese, an denen der Knochen festwachsen soll, mit Apathith beziehungsweise Keramik beschichtet. Im Bereich der den Sattel 1 bildenden Mulde muß auf jede Beschichtung verzichtet werden, da an diesen Stellen ein Anwachsen des Knochens weder beabsichtigt noch erwünscht ist.
Der Schaft 3 kann eine der natürlichen Krümmung des Oberschenkelknochens 4 angepaßte Krümmung aufweisen. Es ist jedoch auch möglich, einen gerade verlaufenden Schaft 3 in einen entsprechenden Oberschenkelknochen 4 einzusetzen. Die Krümmung kann in Richtung der Schwenkebene des Oberschenkelknochens 4 oder quer dazu vorgesehen sein. Auch Zwischenkrümmungen sind denkbar.
Der Sattel 1 kann mit seiner tiefsten Stelle 72 in einem Bereich liegen, der auf einer Verlängerung einer sich durch den Schaft 3 erstreckenden Mittellinie liegt. Je nach der Beschaffenheit des mit der Sattelprothese zu versehenden Beckenknochens 5 ist es jedoch auch möglich, den Sattel 1 gegenüber dieser Mittellinie mehr oder minder stark zu neigen, so daß das durch den Beckenknochen 5 hindurchragende Sattelhorn 7 sich weitgehend dem Beckenknochen 5 zuneigt. In diesem Falle kann zwischen einer Verlängerung 73 der durch das Zylinderstück 2 verlaufenden Mittellinie und einer gedachten Verbindungslinie 77, die zwischen der tiefsten Stelle 72 des Sattelhorns 7 und einem Mittelpunkt des Zylinderstückes 2 im Bereich des Rezeßes 15 verläuft, ein zwischen 0° und 45° liegender Winkel vorgesehen sein, um den sich der Sattel 1 in einer von ihm aufgespannten Ebene in Richtung auf den Beckenknochen 5 neigt. Die jeweils gewünschte Auslenkung des Sattels 1 gegenüber der Mittellinie 74 wird nach der Beschaffenheit des Beckenknochens 5 festgelegt, an den die Sattelprothese angelenkt werden soll. Darüber hinaus kann eine Anpassung an die Form des Beckenknochens 5 auch dadurch geschehen, daß eine Neigung des Sattels 1 gegenüber der Richtung des Schaftes 3 erfolgt, die quer zu einer vom Sattel 1 aufgespannten Ebene verläuft. Dabei kann die Neigung des Sattels 1 in Schwenkrichtung des mit ihm verbundenen Oberschenkelknochens 4 nach vorne oder nach hinten vorgenommen werden. Auf diese Weise kann die Anlenkung des Sattels 1 an einer beliebigen Stelle des Beckenknochens 5 vorgenommen werden, die sich für die Anlenkung des Sattels 1 aus medizinischer Sicht besonders eignet. Schließlich ist es denkbar, den Sattel 1 gegenüber dem Schaft 3 in einer beliebigen Richtung zu neigen, die zwischen einer Richtung liegt, die von der sich durch den Sattel 1 erstreckenden Ebene vorgegeben ist, und einer quer zu dieser Ebene verlaufenden Richtung.

Darüber hinaus kann die Sattelprothese in vielfältiger Weise den jeweils angetroffenen individuellen Verhältnissen angepaßt werden. Beispielsweise ist es denkbar, zwischen dem Zylinderstück 2 und dem Sattel 1 einen Hals 76 vorzusehen, der je nach den individuellen Bedürfnissen mehr oder minder lang ausgebildet ist. Dieser Hals 76 ist entsprechend der Neigung des Sattels 1 mehr oder minder stark geschwungen.

Darüber hinaus ist es möglich, auch den Sattel 1 den jeweiligen Einbauzwecken der Sattelprothese anzupassen. Beispielsweise kann eines der beiden Sattelhörner 7, 8 als ein abgeplattetes Sattelhorn 77 ausgebildet sein, das auf seiner dem anderen Sattelhorn 7 abgewandten Außenseite 78 als eine Ebene ausgebildet ist, die sich im wesentlichen in Längsrichtung des Schaftes 3 erstreckt und planparallel zu einer Ebene verläuft, in der der Oberschenkelknochen 4 seine Schwenkbewegungen ausführt. Dieses abgeplattete Sattelhorn 77 ist auf seiner dem Schaft 3 abgewandten Oberkante 79 mit einer relativ großen Breite versehen, zu der sich die den Sattel ausbildende Mulde 80 sanft erhebt. Diese große Breite der Oberkante 79 dient insbesondere der problemlosen Einordnung des außerhalb des Beckenknochens 75 liegenden Sattelhornes 77 in die von Weichteilen gebildete Umgebung des Beckenknochens 5. Demgegenüber kann das andere Sattelhorn 7 relativ schmal sein und in einer abgerundeten Spitze 81 enden.

Es ist jedoch auch möglich, die zwischen den Sattelhörnern 7, 8 liegende Mulde 80 mit unterschiedlichen Breiten herzustellen. Dabei richtet sich die Breite der Mulde 80 ausschließlich nach der Größe der Öffnung, in die der Sattel 1 mit einem seiner Sattelhörner 7 hindurchragt. Dabei kann die Mulde 80 seitliche Begrenzungen 82, 83 aufweisen, die in Richtung aufeinander zu gewölbt sind und auf diese Weise ein Verengung ausbilden, die als Auflagerfläche für den Beckenknochen 5 dient. Es ist jedoch auch denkbar, die seitlichen Begrenzungen 82, 83 in Richtung voneinander weg zu wölben, so daß die Mulde 80 sich als eine Verbreiterung darstellt, auf der der Beckenknochen 5 aufliegt.

Sowohl durch die Gestaltung des Sattels 1 als auch durch die Länge des Halses 76 beziehungsweise die Länge des Zylinderstückes 2, die zwischen o,1 und 4 cm variieren kann, ist eine sehr weitgehende Anpassung an die individuellen Verhältnisse eines Beckenknochens 5 gewährleistet.

Die Verbindung des Zylinderstückes 2 mit dem Schaft 3 kann mit Hilfe von Konusverbindungen 84, 85 herbeigeführt werden. Dabei ragt beispielsweise der Schaft 3 mit einer in Form eines Konus 86 ausgebildeten Erhöhung in eine Vertiefung 87 hinein, die ebenfalls in Form eines Konus ausgebildet ist und der Gestalt des Konus 86 angepaßt ist. Der Konus 86 trägt an seinem dem Schaft 3 abgewandten Ende eine mit ihm fest verbundene Schraube 88, die mit einem Gewinde eines Sackloches 89 kämmt, das am Ende der Vertiefung 87 vorgesehen ist. Durch Verschrauben der Schraube 88 im Sackloch 89 wird der Konus 86 fest in die Vertiefung 87 hineingezogen, so daß eine formschlüssige Verbindung zwischen dem Zylinderstück 2 und dem Schaft 3 zustande kommt. Ähnliche Konusverbindungen 84, 85 sind auch zwischen den einzelnen Abstandsringen 24 sowie zwischen diesen und dem Kragen 17 möglich. Darüber hinaus können Konusverbindungen 84, 85 auch zwischen den Distanzringen 55, 56, sowie zwischen diesen und dem Zylinderstück 2 und dem Kragen 17 andererseits vorgesehen sein.

Der Schaft 3 kann unmittelbar im Oberschenkelknochen 4 in der bereits besprochenen Weise verankert werden. Es ist jedoch auch möglich, den Schaft 3 in eine Totalfemurprothese einmünden zu lassen, die einerseits den in dem Beckenknochen 5 gelagerten Sattel 1 und andererseits eine Kniegelenkendoprothese aufweist. In diesem Falle ersetzt die Totalfemurprothese den gesamten Oberschenkelknochen 4.

DIPL.-ING. DR. GERT HELDT

Rechtsanwalt, Patentanwalt

Dr. Gert Heldt - Neuer Wall 57 ᴵⱽ - 2000 Hamburg 36

Neuer Wall 57 ᴵⱽ - 2000 Hamburg 36
Ruf 040 / 37 15 77 + 37 17 49

Sprechstunden nach Vereinbarung

Telegramme Patentheidt, Hamburg

Anwaltsakte: EN 29

GMT Gesellschaft für

medizinische Technik mbH

Holstenstraße 2

2ooo Hamburg 5o

Waldemar Link GmbH & Co.

Barkhausenweg 1o

2ooo Hamburg 63

S a t t e l p r o t h e s e

### Patentansprüche:

1. Sattelprothese zum Ersatz eines an einem Beckenknochen auszubildenden das Hüftgelenk ersetzenden Gelenkes mit zwei einen Sattel zwischen sich ausbildenden Sattelhörnern, die sich im Bereich des Sattels zu einem sich in Längsrichtung erstreckenden Zylinderstück vereinigen, das in einen in einen Oberschenkelknochen einzusetzenden Schaft einmündet, zwischen dem und dem Zylinderstück ein Kragen ausgebildet ist, der sich auf einem dem Sattel zugewandten oberen Ende des Oberschenkelknochens abstützt, dadurch gekennzeichnet, daß der Sattel (1) in seiner Lage zwischen dem oberen Abschluß (19) des Oberschenkelknochens (4) einerseits und dem Beckenknochen (5) andererseits einstellbar angeordnet ist.

2. Sattelprothese nach Anspruch 1, dadurch gekennzeichnet, daß das Zylinderstück (2) und der Schaft (3) als zwei voneinander getrennte Bauteile ausgebildet sind, die gegeneinander einstellbar angeordnet sind.

3. Sattelprothese nach Anspruch 1 und 2, dadurch gekennzeichnet, daß am Zylinderstück (2) ein sich in Richtung des Schaftes (3) erstreckender Bolzen (12) befestigt ist, der in eine entsprechende Bohrung (13) hineinragt, die sich in Längsrichtung des Schaftes (3) durch diesen erstreckt.

4. Sattelprothese nach Anspruch 1 bis 3, dadurch gekennzeichnet, daß der Bolzen (12) in voneinander verschiedenen Schwenkstellungen im Schaft (3) justierbar angeordnet ist.

5. Sattelprothese nach Anspruch 1 bis 4, dadurch gekennzeichnet, daß sich quer zur Längsrichtung des Bolzens (12) mindestens eine Querrille (2o) durch dessen Oberfläche erstreckt, in die eine sich quer zu dessen Längsrichtung durch den Schaft (3) erstreckende Madenschraube (21) hineinragt.

6. Sattelprothese nach Anspruch 1 bis 5, dadurch gekennzeichnet, daß mindestens zwei einander parallel verlaufende Querrillen (2o) sich im Abstand voneinander durch die Oberfläche des Bolzens (12) erstrecken.

7. Sattelprothese nach Anspruch 1 bis 6, dadurch gekennzeichnet, daß sich der Schaft (3) durch Abstandsringe (24) erstreckt, von denen mindestens einer zwischen dem oberen Abschluß (19) des Oberschenkelknochens (4) einerseits und einer ihm zugewandten Unterfläche (18) des Kragens (17) andererseits angeordnet ist.

8. Sattelprothese nach Anspruch 1 bis 7, dadurch gekennzeichnet, daß auf dem oberen Abschluß (19) des Oberschenkelknochens (4) eine Druckscheibe angeordnet ist, auf der sich der Abstandsring (24) abstützt.

9. Sattelprothese nach Anspruch 1 bis 9, dadurch gekennzeichnet, daß die Druckscheibe als Metallring (28) ausgebildet ist.

1Q Sattelprothese nach Anspruch 1 bis 9 dadurch gekennzeichnet, daß der Kragen (17) eine asymmetrische Ausbildung aufweist, die im Bereich des Trochanters Major (32) eine schmalere Unterfläche (18) als im Bereich des übrigen Umfangs des Kragens (17) aufweist.

11. Sattelprothese nach Anspruch 1 bis 1q dadurch gekennzeichnet, daß am Kragen (17) eine sich quer zur Längsrichtung des Bolzens (12) erstreckende Halterung (36) befestigt ist, die von Muskeln (31) durchwachsen ist.

12. Sattelprothese nach 11, dadurch gekennzeichnet, daß die Halterung (36) als eine Trochanterplatte (38) ausgebildet ist, die eine Ebene aufspannt, die sich im wesentlichen parallel zu einer durch den Bolzen (12) verlaufenden Mittellinie erstreckt.

13. Sattelprothese nach Anspruch 11 und 12, dadurch gekennzeichnet, daß sich durch die Trochanterplatte (33) eine Vielzahl von Verklammerungsbohrungen (44) erstreckt, die von Muskeln (31) durchdrungen sind.

14 Sattelprothese nach Anspruch 11 bis 13, dadurch gekennzeichnet, daß die Trochanterplatte (38) mit einer Schraube (45) am Kragen (17) befestigt ist, deren Längsachse sich quer zur Ebene der Trochanterplatte (38) erstreckt.

15. Sattelprothese nach Anspruch 11 bis 14, dadurch gekennzeichnet, daß die Trochanterplatte (38) eine leichte Wölbung in Form einer Teilfläche eines Zylinders aufweist, dessen Mittelachse parallel zur Mittelachse des Bolzens (12) verläuft.

16. Sattelprothese nach Anspruch 1 bis 15, dadurch gekennzeichnet, daß der Kragen (17) lösbar auf dem Bolzen (12) befestigt ist.

17. Sattelprothese nach Anspruch 16, dadurch gekennzeichnet, daß der Kragen (17) eine Bohrung (53) aufweist, durch die der Bolzen (12) hindurchragt.

18 . Sattelprothese nach Anspruch 16 und 17 , dadurch gekennzeichnet, daß durch den Kragen (17) in Richtung auf den Bolzen (12) eine Befestigungsschraube hindurchragt, die sich in einer vom Kragen (17) aufgespannten Ebene erstreckt.

19. Sattelprothese nach Anspruch 16 bis 18 , dadurch gekennzeichnet, daß die Schraube eine in Richtung auf den Bolzen (12) ragende Spitze aufweist, die mit dem Bolzen (12) in Formschluß ist.

20 . Sattelprothese nach Anspruch 16 bis 19 , dadurch gekennzeichnet, daß in der Oberfläche des Bolzens (12) mindestens eine die Spitze der Schraube aufnehmende Bohrung vorgesehen ist.

21 . Sattelprothese nach Anspruch 16 bis 19 , dadurch gekennzeichnet, daß in der Oberfläche des Bolzens (12) mindestens eine sich quer zur Längsrichtung des Bolzens (12) erstreckende Querrille (20) ausgebildet ist, in die die Spitze der Schraube hineinragt.

22. Sattelprothese nach Anspruch 16 bis 21, dadurch gekennzeichnet, daß der Kragen (17) eine dem Bolzen (12) abgewandte äußere Oberfläche in Form eines Zylinders aufweist.

23. Sattelprothese nach Anspruch 16 bis 22, dadurch gekennzeichnet, daß der Kragen (17) eine dem Bolzen (12) abgewandte äußere Oberfläche aufweist, die an ihrem dem Oberschenkelknochen (4) abgewandten oberen Ende zylindrisch ausgebildet ist und sich im Bereich seiner Unterfläche (18) in Form eines Wulstes (30) aufweitet.

24. Sattelprothese nach Anspruch 16 bis 23, dadurch gekennzeichnet, daß der Kragen (17) eine einem jeweiligen Verwendungszweck entsprechende in Richtung des Bolzens (12) verlaufende Höhe aufweist.

25. Sattelprothese nach Anspruch 16 bis 24, dadurch gekennzeichnet, daß der Kragen (17) im Bereich seiner Oberfläche mit dem Zylinderstück (2) verankert ist.

26. Sattelprothese nach Anspruch 16 bis 25, dadurch gekennzeichnet, daß am Kragen (17) ein in Richtung auf das Zylinderstück (2) ragender Zapfen (48) vorgesehen ist, der in eine entsprechende Ausnehmung (54) hineinragt, die im Zylinderstück (2) vorgesehen ist.

27. Sattelprothese nach Anspruch 16 bis 26, dadurch gekennzeichnet, daß die Ausnehmung (54) sich in einer zylindrischen Oberfläche des Zylinderstückes (2) erstreckt.

28 . Sattelprothese nach Anspruch 16 bis 27., dadurch gekennzeichnet, daß der Zapfen (48) als Zylinder ausgebildet ist, dessen unteres Ende (51) in den Kragen (17) und dessen oberes Ende (49) in das Zylinderstück (2) hineinragt, dessen Ausnehmung (54) als Hohlzylinder ausgebildet ist, in dem der Zylinder mit einer engen Passung geführt ist.

29. Sattelprothese nach Anspruch 16 bis 26, dadurch gekennzeichnet, daß der Zylinder jeweils nur mit einer Hälfte seiner im Bereich sowohl des oberen als auch des unteren Endes (51) vorgesehenen Oberfläche in das Zylinderstück (2) und den Kragen (17) hineinragt.

30. Sattelprothese nach Anspruch 16 bis 29 dadurch gekennzeichnet, daß der Kragen (17) im Bereich seiner Oberfläche mit dem Schaft (3) verankert ist.

31. Sattelprothese nach Anspruch 16 bis 30 , dadurch gekennzeichnet, daß am Schaft (3) ein in Richtung auf den Kragen (17) ragender Zapfen (48) vorgesehen ist, der in eine entsprechende Vertiefung hineinragt, die am Kragen (17) vorgesehen ist.

32. Sattelprothese nach Anspruch 16 bis 31 , dadurch gekennzeichnet, daß der Zapfen (48) als Zylinder ausgebildet ist, dessen unteres Ende (51) in den Schaft und dessen oberes Ende (49) in den Kragen (17)    hineinragt, dessen Ausnehmung (54) als Hohlzylinder ausgebildet ist, in dem der Zylinder mit enger Passung geführt ist.

33. Sattelprothese nach Anspruch 16 bis 32, dadurch gekennzeichnet, daß der Zylinder jeweils nur mit einer Hälfte seiner im Bereich sowohl des oberen als auch des unteren Endes (51) vorgesehenen Oberfläche in den Kragen (17) und den Schaft (3) hineinragt.

34. Sattelprothese nach Anspruch 16 bis 20, dadurch gekennzeichnet, daß der Kragen (17) im Bereich seiner Oberfläche mit einem unmittelbar an ihm anliegenden Abstandsring (24) verankert ist.

35. Sattelprothese nach Anspruch 34, dadurch gekennzeichnet, daß am Abstandsring (24) ein in Richtung auf den Kragen (17) ragender Zapfen (48) vorgesehen ist, der in eine entsprechende Ausnehmung (54) hineinragt, die am Kragen (17) vorgesehen ist.

36. Sattelprothese nach Anspruch 34 und 35, dadurch gekennzeichnet, daß der Zapfen (48) als Zylinder ausgebildet ist, dessen unteres Ende (51) in den Abstandsring (24) und dessen oberes Ende (49) in den Kragen (17) hineinragt, dessen Ausnehmung (54) als Hohlzylinder ausgebildet ist, in dem der Zylinder mit enger Passung geführt ist.

37. Sattelprothese nach Anspruch 34 bis 36, dadurch gekennzeichnet, daß der Zylinder jeweils nur mit einer Hälfte seiner im Bereich sowohl des oberen als auch des unteren Endes (51) vorgesehenen Oberfläche in den Kragen (17) und den Abstandsring (24) hineinragt.

38. Sattelprothese nach Anspruch 16 bis 37, dadurch gekennzeichnet, daß das Zylinderstück (2) als mindestens ein Distanzring (55, 56, 63, 64) ausgebildet ist, der auf den Bolzen (12) aufgeschoben ist, und sich in einem Bereich erstreckt, der zwischen einer dem Sattel (1) zugewandten Oberfläche (50) des Kragens (17) und dem Sattel (1) liegt.

39 Sattelprothese nach Anspruch 1b bis 3?, dadurch gekennzeichnet, daß der Distanzring (55, 56, 63, 64) sich an einem Rezess (15) abstützt, der im Bereich des Zylinderstückes (2) ausgebildet ist und auf dem sich der Bolzen (12) erhebt.

40. Sattelprothese nach Anspruch 16 bis 39, dadurch gekennzeichnet, daß der Distanzring (55, 56, 63, 64) sowohl dem Rezess (15) als auch dem Kragen (17) zugewandte glatte Oberflächen aufweist.

41. Sattelprothese nach Anspruch 16 bis 39 dadurch gekennzeichnet, daß der Distanzring (55, 56, 63, 64) mindestens auf einer seiner beiden dem Kragen (17) einerseits und dem Rezeß (15) andererseits zugewandten Oberflächeneine Zähnelung (6o) aufweist, deren Form und Verlauf einer ihr zugewandten Zähnelung (61) entspricht.

42. Sattelprothese nach Anspruch16 bis 41, dadurch gekennzeichnet, daß an einem der Distanzringe (55, 56, 63, 64) eine sich quer zur Längsrichtung des Bolzens (12) erstreckende Halterung (36) zur Anlenkung von Muskeln (31) befestigt ist.

43. Sattelprothese nach Anspruch 16 bis 42, dadurch gekennzeichnet, daß die Halterung (36) als eine Trochanterplatte (38) ausgebildet ist, die eine Ebene aufspannt, die sich im wesentlichen in Richtung einer durch den Bolzen (12) verlaufenden Mittelachse erstreckt.

44. Sattelprothese nach Anspruch 16 bis 43, dadurch gekennzeichnet, daß die Trochanterplatte (38) von einer Vielzahl von Verklammerungsbohrungen (44) durchdrungen ist, die von Muskeln (31) durchwachsen sind.

45. Sattelprothese nach Anspruch 16 bis 44, dadurch gekennzeichnet, daß die Trochanterplatte (38) mit einer Schraube (45) an dem Distanzring (55, 56, 63, 64) befestigt ist, deren Längsrichtung sich quer zur Ebene der Trochanterplatte (38) erstreckt.

46. Sattelprothese nach Anspruch 16 bis 45, dadurch gekennzeichnet, daß die Trochanterplatte (38) eine leichte Wölbung in Form einer Teilfläche eines Zylinders aufweist, dessen Mittelachse parallel zur Mittelachse des Bolzens (12) verläuft.

47. Sattelprothese nach Anspruch 16 bis 46, dadurch gekennzeichnet, daß der Kragen (17) und der Distanzring (55, 56, 63, 64) im Bereich ihrer Oberflächen miteinander verankert sind.

48. Sattelprothese nach Anspruch 16 bis 47, dadurch gekennzeichnet, daß am Kragen (17) ein in Richtung auf den Distanzring (55, 56, 63, 64) ragender Zapfen (48) vorgesehen ist, der in eine entsprechende Ausnehmung (54) hineinragt, die am Distanzring (55,56,63, 64) vorgesehen ist.

49. Sattelprothese nach Anspruch 16 bis 48, dadurch gekennzeichnet, daß die Ausnehmung (54) sich in einer zylinderischen Oberfläche des Distanzringes (55, 56, 63, 64) erstreckt.

Anspruch/Ansprüche Nr. 44-46, 48, 49
gilt/gelten als aufgegeben

50. Sattelprothese nach Anspruch 16 bis 49, dadurch gekennzeichnet, daß der Zapfen (48) als Zylinder ausgebildet ist, dessen unteres Ende (51) in den Kragen (17) und dessen oberes Ende (49) in den Distanzring (55, 56, 63, 64) hineinragt, dessen Ausnehmung (54) als Hohlzylinder ausgebildet ist, in dem der Zylinder mit enger Passung geführt ist.

51. Sattelprothese nach Anspruch 16 bis 5o, dadurch gekennzeichnet, daß der Zylinder jeweils nur mit einer Hälfte seiner im Bereich sowohl des oberen als auch des unteren Endes (51) vorgesehenen Oberfläche in den Distanzring (55, 56, 63, 64) und den Kragen (17) hineinragt.

52 Sattelprothese nach Anspruch 16 bis 51, dadurch gekennzeichnet, daß zwei einander benachbarte Distanzringe (55, 56, 63, 64) im Bereich ihrer Oberflächen miteinander verankert sind.

53. Sattelprothese nach Anspruch 16 bis 52, dadurch gekennzeichnet, daß an einem Distanzring (55) ein in Richtung auf den benachbarten Distanzring (56) ragender Zapfen (48) vorgesehen ist, der in eine entsprechende Ausnehmung (54) hineinragt, die am benachbarten Distanzring (56) vorgesehen ist.

54. Sattelprothese nach Anspruch 16 bis 53, dadurch gekennzeichnet, daß die Ausnehmung (54) sich in einer zylindrischen Oberfläche des Distanzringes (56) erstreckt.

55. Sattelprothese nach Anspruch 16 bis 54, dadurch gekennzeichnet, daß der Zapfen (48) als Zylinder ausgebildet ist, der jeweils zur Hälfte in die beiden einander benachbarten Distanzringe (55, 56) hineinragt und die Ausnehmung (54) als Hohlzylinder ausge-

0228511

bildet ist, in dem der Zylinder mit einer engen Passung geführt ist.

56. Sattelprothese nach Anspruch 16 bis 55, dadurch gekennzeichnet, daß der Zylinder jeweils nur mit einer Hälfte seiner im Bereich sowohl der oberen als auch der unteren Hälfte vorgesehenen Oberfläche in die Distanzringe (55, 56) hineinragt.

57. Sattelprothese nach Anspruch 16 bis 56, dadurch gekennzeichnet, daß die Distanzringe (55, 56, 63, 64) mit einander zugewandten Zähnelungen (6o, 61) auf ihren Oberflächen versehen sind.

58. Sattelprothese nach Anspruch 16 bis 56, dadurch gekennzeichnet, daß die Distanzringe (55, 56, 63, 64), die zwischen dem Rezeß (15) und dem mit der Trochanterplatte (38) versehenen Distanzring (55) vorgesehen sind, mit einander zugewandten Zähnelungen (6o, 61) versehen sind, während die zwischen dem Kragen (17) und dem mit der Trochanterplatte (38) versehenen Distanzring (55) liegenden Distanzringe (55) einander zugewandte glatte Oberflächen (5o) aufweisen.

59. Sattelprothese nach Anspruch 1 bis 58, dadurch gekennzeichnet, daß der Sattel (1) an seiner tiefsten Stelle (72) einen Versatz aufweist, bei dem je nach Einbauzweck zwischen einer Verlängerung der durch das Zylinderstück (2) verlaufenden Mittellinie (73) und einer gedachten zwischen der tiefsten Stelle (72) des Sattels (1) und dem Mittelpunkt einer das Zylinderstück (2) an seinem oberen Rand begrenzenden kreisförmigen Schnittebene verlaufenden Verbindungslinie (75) ein Winkel zwischen 0° und 45° liegt.

60. Sattelprothese nach Anspruch 59, dadurch gekennzeichnet, daß der Versatz in einer vom Sattel (1) aufgespannten Ebene vorgesehen ist und zwischen dem Zylinderstück (2) und der tiefsten Stelle (72) eine in Form eines Halses (76) ausgebildete einseitige Krümmung angeordnet ist.

61. Sattelprothese nach Anspruch 59 und 6o, dadurch gekennzeichnet, daß der Versatz quer zu einer vom Sattel (1) aufgespannten Ebene in Richtung eines vorwärts schwingenden Oberschenkels vorgesehen ist.

62. Sattelprothese nach Anspruch 59 und 6o, dadurch gekennzeichnet, daß der Versatz quer zu einer vom Sattel (1) aufgespannten Ebene in Richtung eines rückwärts schwingenden Oberschenkels vorgesehen ist.

63. Sattelprothese nach Anspruch 1 bis 62, dadurch gekennzeichnet, daß das Zylinderstück (2) je nach Einbauzweck eine Länge von o,1 bis 4 cm aufweist.

64. Sattelprothese nach Anspruch 1 bis 63, dadurch gekennzeichnet, daß eines der beiden Sattelhörner (7, 8) eine als Abplattung ausgebildete äußere Begrenzung (9, 1o) aufweist, deren Ebene planparallel zu einer Schwenkebene des Oberschenkelknochens (4) verläuft.

65. Sattelprothese nach Anspruch 64, dadurch gekennzeichnet, daß die Ebene der Abplattung sich in etwa in Längsrichtung des Schaftes (3) erstreckt, aus der das andere Sattelhorn (8) herausragt.

66. Sattelprothese nach Anspruch 1 bis 65, dadurch gekennzeichnet, daß eines der beiden Sattelhörner (7) an seinem der tiefsten Stelle (72) abgewandten Ende eine sich in der Schwenkrichtung des Oberschenkelknochens (4) erstreckende Breite aufweist, während das

Anspruch/Ansprüche Nr.6o-66
gilt/gelten als aufgegeben

andere Sattelhorn (8) in einer abgerundeten Spitze
(81) endet.

67. Sattelprothese nach Anspruch 1 bis **66**, dadurch gekennzeichnet, daß eine den Sattel (1) bildende Mulde
(8o) seitliche Begrenzungen (82, 83) aufweist, die in
einem zwischen den beiden Sattelhörnern (7, 8) liegenden Bereich in Richtung aufeinander zu gewölbt sind
und als Verengung einer von der Mulde (8o) ausgebildeten Auflagerfläche ausgebildet sind.

68. Sattelprothese nach Anspruch 1 bis 66, dadurch gekennzeichnet, daß eine den Sattel (1) bildende Mulde
(8o) seitliche Begrenzungen (82, 33) aufweist, die in
einem zwischen den beiden Sattelhörnern (7, 8) liegenden Bereich in Richtung voneinander weg gewölbt sind
und als Verbreiterung einer von der Mulde (8o) ausgebildeten Auflagerfläche ausgebildet sind.

69. Sattelprothese nach Anspruch 1 bis 68, dadurch gekennzeichnet, daß der Schaft (3) eine anatomische
Krümmung aufweist.

7o. Sattelprothese nach Anspruch 1 bis 69, dadurch gekennzeichnet, daß die Krümmung in einer durch den Sattel (1) gelegten Mittelebene verläuft.

71. Sattelprothese nach Anspruch 1 bis 7o, dadurch gekennzeichnet, daß die Krümmung in einer quer zur Mittelebene des Sattels (1) verlaufenden Ebene vorgesehen ist.

72. Sattelprothese nach Anspruch 1 bis 71, dadurch gekennzeichnet, daß die Krümmung in mindestens einer von
einer Schar von Ebenen verläuft, die sich zwischen der durch den Sattel (1) gelegten Mittelebene und einer quer zur Mittelebene des Sattels (1) verlaufenden Ebene erstrecken.

73. Sattelprothese nach Anspruch 1 bis 72, dadurch gekennzeichnet, daß der Schaft (3) in eine den Oberschenkelknochen (4) und ein sich daran anschliessendes Kniegelenk ersetzende Totalprothese einmündet.

74. Sattelprothese nach Anspruch 1 bis 73, dadurch gekennzeichnet, daß der Schaft (3) eine glatte Oberfläche (65) aufweist.

75. Sattelprothese nach Anspruch 1 bis 73, dadurch gekennzeichnet, daß der Schaft (3) eine gerillte Oberfläche (68) aufweist, deren Rillen (7o) sich in Längsrichtung des Schaftes (3) erstrecken und bei dem zwischen je zwei einander benachbarten Rillen (7o) eine sich in den Oberschenkelknochen (4) einschneidende scharfe Kante (71) vorgesehen ist.

76. Sattelprothese nach Anspruch 1 bis 73, dadurch gekennzeichnet, daß der Schaft (3) mit einer Vielzahl kleiner Erhebungen auf seiner Oberfläche versehen ist.

77. Sattelprothese nach Anspruch 76, dadurch gekennzeichnet, daß die kleinen Erhebungen als Kugeln ausgebildet sind, die fest mit der Oberfläche verbunden sind.

78. Sattelprothese nach Anspruch 1 bis 77, dadurch gekennzeichnet, daß der Schaft (3) eine poröse Oberfläche aufweist.

0228511

79. Sattelprothese nach Anspruch 1 bis 78, dadurch gekennzeichnet, daß der Schaft (3) einen kreisförmigen Querschnitt aufweist.

8o. Sattelprothese nach Anspruch 1 bis 78, dadurch gekennzeichnet, daß der Schaft (3) einen eckigen Querschnitt hat, dessen Ecken abgerundet sind.

81. Sattelprothese nach Anspruch 8o, dadurch gekennzeichnet, daß der Schaft (3) einen dreieckigen Querschnitt aufweist.

82. Sattelprothese nach Anspruch 8o, dadurch gekennzeichnet, daß der Schaft (3) einenviereckigen Querschnitt aufweist.

83. Sattelprothese nach Anspruch 1 bis 82, dadurch gekennzeichnet, daß sie außerhalb ihrer als Gelenk dienenden Oberfläche mit einem bioaktiven Material beschichtet ist.

84. Sattelprothese nach Anspruch 83, dadurch gekennzeichnet, daß sie in ihren einem Bindegewebe benachbarten Teilen mit einem Polyurethanschaum beschichtet ist.

85. Sattelprothese nach Anspruch 83, dadurch gekennzeichnet, daß sie in ihren einem Bindegewebe benachbarten Teilen mit Velour beschichtet beschichtet ist.

86. Sattelprothese nach Anspruch 83 bis 85, dadurch gekennzeichnet, daß sie in ihren einem Knochen benachbarten Teilen mit Apathith beschichtet ist.

87. Sattelprothese nach Anspruch 83 bis 85, dadurch gekennzeichnet, daß sie in ihren einem Knochen benachbarten Teilen mit Keramik beschichtet ist.

Anspruch/Ansprüche Nr. 79-82; 84-87 gilt/gelten als aufgegeben

88. Sattelprothese nach Anspruch 1 bis 87, dadurch gekennzeichnet, daß die Abstandsringe (24) aus Metall bestehen.

89. Sattelprothese nach Anspruch 1 bis 87, dadurch gekennzeichnet, daß die Abstandsringe (24) aus Polyäthylen bestehen.

9o. Sattelprothese nach Anspruch 1 bis 87, dadurch gekennzeichnet, daß die Abstandsringe (24) aus Apathith bestehen.

91. Sattelprothese nach Anspruch 1 bis 87, dadurch gekennzeichnet, daß die Abstandsringe (24) aus Keramik bestehen.

92. Sattelprothese nach Anspruch 1 bis 91, dadurch gekennzeichnet, daß die Distanzringe (55, 56) aus Metall bestehen.

93. Sattelprothese nach Anspruch 1 bis 91, dadurch gekennzeichnet, daß die Distanzringe (55, 56) aus Polyäthylen bestehen.

94. Sattelprothese nach Anspruch 1 bis 91, dadurch gekennzeichnet, daß die Distanzringe (55, 56) aus Apathith bestehen.

95. Sattelprothese nach Anspruch 1 bis 91, dadurch gekennzeichnet, daß die Distanzringe (55, 56) aus Keramik bestehen.

96. Sattelprothese nach Anspruch 1 bis 95, dadurch gekennzeichnet, daß das Zylinderstück (22) in einem in Richtung auf den Schaft (3) gerichteten Konus (86) endet, der in einen im Schaft (3) ausgebildeten Innenkonus mündet und in diesem befestigt ist.

97. Sattelprothese nach Anspruch 96, dadurch gekennzeichnet, daß der Konus (86) mit einer aus ihm in Richtung auf den Innenkonus herausragenden Schraube (88) an diesem befestigt ist.

98. Sattelprothese nach Anspruch 96 und 97, dadurch gekennzeichnet, daß die Schraube (88) aus einer in Richtung auf den Innenkonus gerichteten Abschlußfläche aus dem Konus (86) herausragt und mit einem sich durch den Innenkonus erstreckenden Gewinde verschraubt ist.

99. Sattelprothese nach Anspruch 96 bis 98, dadurch gekennzeichnet, daß jeder Distanzring (55, 56) auf seiner dem Schaft (3) zugewandten Unterseite mit einem in Richtung auf den Schaft (3) ragenden Konus (86) versehen ist, in dem ein von einem benachbarten Konus beaufschlagter Innenkonus ausgebildet ist.

1oo. Sattelprothese nach Anspruch 99, dadurch gekennzeichnet, daß zwischen den Konen (86) der Distanzringe (55, 56) Schraubverbindungen vorgesehen sind.

1o1. Sattelprothese nach Anspruch 96 bis 1oo, dadurch gekennzeichnet, daß jeder Abstandsring (24) auf seiner dem Schaft (3) zugewandten Unterseite mit einem in Richtung auf den Schaft (3) ragenden Konus (86) versehen ist, in dem ein von einem benachbarten Konus (86) beaufschlagter Innenkonus ausgebildet ist.

1o2. Sattelprothese nach Anspruch 1o1, dadurch gekennzeichnet, daß zwischen denen Konen (86) der Distanzringe (55, 56) Schraubverbindungen vorgesehen sind.

Fig. 2

9

11

10

15

17

19

22

20

31

32

13

4

3

V V

Fig. 5

65

66

67

4

Fig.3

Fig.6

72

7

73

75

74

2

41

43

38

48

45

51

47

36

Fig.9

63

62

Fig. 7

Fig. 8

0228511

Fig. 10

Fig. 11

Fig. 12

Fig. 15

Fig. 16

0228511

Fig. 14

Fig. 13

43

44

42

40

44    43

41

42

40   38   39

Fig. 17

33    17    34

Fig. 18

Fig. 19

*Fig. 20*

*Fig. 21*

*Fig. 22*

Fig. 23

1

2

87

84

89

88

84

85

3